# EUROPEAN PATENT APPLICATION

(11) **EP 2 311 498 A1**
(43) Date of publication of application: **20.04.2011**
(21) Application number: 09804706.1
(22) Date of filing: 03.08.2009
(51) Int. Cl.: A61K 47/12, A61K 9/08, A61K 47/10, A61K 47/14

(54) **EXTERNAL PREPARATION COMPRISING FATTY ACID SALT OR BENZOIC ACID SALT OF BASIC PHARMACOLOGICALLY ACTIVE COMPONENT, AND METHOD FOR PRODUCTION THEREOF**

(30) Priority: 05.08.2008 JP 2008202301; 10.11.2008 JP 2008287627
(71) Applicant: Medrx Co., Ltd., Kagawa 769-2712 (JP)
(72) Inventor: HANMA, Noritaka, Higashikagawa-shi Kagawa 769-2712 (JP); ISHIBASHI, Masaki, Higashikagawa-shi Kagawa 769-2712 (JP); HAMAMOTO, Hidetoshi, Higashikagawa-shi Kagawa 769-2712 (JP); YAMANAKA, Katsuhiro, Higashikagawa-shi Kagawa 769-2712 (JP)
(74) Representative: Müller-Boré & Partner Patentanwälte
(86) International application number: PCT/JP2009/003673
(87) International publication number: WO 2010/016219

(57) **Abstract**

It has been demanded to develop a formulation for a novel external preparation having excellent transdermal absorption of a basic pharmacologically active component contained therein. It is found that the lipid solubility (logP values) of a basic pharmacologically active component and an organic acid (particularly a fatty acid) contribute significantly to the transdermal absorption of the basic pharmacologically active component in the selection of proper combinations of the basic pharmacologically active component and the organic acid. Namely, it is found that superior transdermal absorption of a basic pharmacologically active component having a lipid solubility level of 0.5 to 5 can be achieved by forming a salt of the component with a fatty acid having a lipid solubility level of -1 to 4. Thus, it becomes possible to provide a novel external preparation having excellent transdermal absorption properties.

## Description

### TECHNICAL FIELD

This invention relates to an external preparation comprising a salt such as a fatty acid salt of a basic pharmacologically active component having the ratio of 1:1 of the component to the acid as well as a method for production thereof. In particular, the present invention relates to a novel ionic liquid comprising a fatty acid salt or a benzoic acid salt of a basic pharmacologically active component such as morphine, lidocaine, tramadol and pentazocine and an external preparation comprising the liquid.

### BACKGROUND ART

It has been known from Nonpatent document 1 and Patent document 1 that a transdermal absorbability of a basic pharmacologically active component is improved by forming a salt with a fatty acid or a bile acid. In addition, an acetic acid salt, a lactic acid salt and a benzoic acid salt of risperidone, which is a basic pharmacologically active component, are described in Patent document 2 and it is reported that transdermal absorption of risperidone is increased by depression of the melting point which is caused as a result of forming the salt.
Likewise, it is reported in Nonpatent document 2 that as a result of forming a fatty acid salt (ionic pair) of propranolol, which is a basic pharmacologically active component, for alteration of the fatty acid, the lipid solubility of the salt can be highly improved. It is also shown that a transdermal absorbability of the pharmacologically active component is improved in consequence of the improvement of the lipid solubility of the salt.

On the other hand, it is described in Patent document 2 that a risperidone salt showing more significant depression of the melting point has a better transdermal absorbability. Comparing an acetic acid salt to a benzoic acid salt, it may be thought that the benzoic acid salt has higher lipid solubility than the acetic acid salt. However, the benzoic acid salt has a less improved transdermal absorbability than the acetic acid salt.
Thus, with regard to the transdermal absorbability of a salt such as a fatty acid salt of a basic pharmacologically active component, it was not well clarified what a major contributing factor is. Therefore, as shown in Patent document 3, various organic acids including acetic acid have been used as an absorption promoter in a transdermal absorption preparation of a basic pharmacologically active component.

Also, as shown in Patent document 2, it has generally been thought that it was preferable to use an excess amount of an organic acid because an external preparation has an excellent transdermal absorbability of a basic pharmacologically active component when the organic acid was used in more than an equimolar amount (for example, 2-3 times) to the basic pharmacologically active component.
However, the actual condition of a transdermal absorbability of a salt such as a fatty acid salt of a basic pharmacologically active component has not been reviewed well.

### PRIOR ART

### PATENT DOCUMENT(S)

[Patent document 1] JP-A-2001-508027
[Patent document 2] JP-A-2006-169238
[Patent document 3] WO 01/007018

### NONPATENT DOCUMENT(S)

[Nonpatent document 1] FRAGRANCE JOURNAL, 1998-9, 71-78
[Nonpatent document 2] Pharmacokinetics, Vol.10, S60-S63 (1995)

### DISCLOSURE OF INVENTION

### PROBLEM TO BE SOLVED

This invention provides an nonaqueous external preparation having a excellent transdermal absorbability which comprises a salt compound such as a fatty acid salt or a benzoic acid salt of a basic pharmacologically active component having the ratio of 1:1 of the component (for example, morphine, lidocaine, pentazocine and amitriptyline) to the acid. In addition, an object of the present invention is to provide a method for production of the external preparation.

### MEANS FOR SOLVING PROBLEM

The present inventors prepared an equimolecular salt of a basic pharmacologically active component and a fatty acid, and then, studied a correlation between logP of the fatty acid and a transdermal absorbability of the basic pharmacological preparation. That is, the present inventors found that a nonaqueous external preparation having an excellent transdermal absorbability could be produced by forming a salt consisting of a compound having a logP of 0.5 to 5 as a basic pharmacologically active component and a compound having a logP of - 1 to 4 as an organic acid (for example, a fatty acid or a benzoic acid). In addition, the present inventors found that an external preparation containing a high concentration (i.e., 20 - 30 %) of an active component having high preservation stability without precipitation of the active component could be obtained by using these organic acid salts. For example, in the case of a lidocaine preparation, there is no precipitation of crystals of lidocaine on an adhesion layer of a tape preparation for transdermal absorption containing a high concentration (45 %) of lidocaine. In addition, a transdermal absorbability of a basic pharmacological agent could be further improved by studying a suitable solvent composition as well as a combination of an external preparation base used for the organic acid salt of the present invention, other than a combination of the above mentioned logPs. The present inventors completed the present invention based on these results.

That is, the subject matters of the present invention are as follows:
(1) A nonaqueous external preparation having an excellent transdermal absorbability comprising an organic acid salt of a basic pharmacologically active component as an active component characterized by comprising:
   an equimolar salt consisting of a compound having a logP of 0.5 to 5 as a basic pharmacologically active component and a compound having a logP of -1 to 4 as an organic acid; and
   an ester solvent and/or an alcohol solvent.
(2) The nonaqueous external preparation according to the above item (1) wherein the basic pharmacologically active component is one or more selected from the group consisting of tramadol, lidocaine, pentazocine, tolperisone, eperisone, amitriptyline, imipramine, donepezil and morphine.
(3) The nonaqueous external preparation according to the above item (1) wherein the basic pharmacologically active component is a compound having the logP of 2 to 5.
(4) The nonaqueous external preparation according to any of the above items (1) to (3) wherein the organic acid is selected from the group consisting of acetic acid, propionic acid, butyric acid, hexanoic acid, glycolic acid, methoxyacetic acid, lactic acid, levulinic acid, benzoic acid, salicylic acid, acetylsalicylic acid and 3-hydroxybutyric acid.
(5) The nonaqueous external preparation according to any of the above items (1) to (4) wherein the organic acid is a compound having the logP of -1 to 2.
(6) The nonaqueous external preparation according to any of the above items (1) to (5) wherein the ester solvent is one or more selected from the group consisting of isopropyl myristate, diethyl sebacate, medium-chain triglyceride and propylene carbonate.
(7) The nonaqueous external preparation according to any of the above items (1) to (6) wherein the alcohol solvent is one or more selected from the group consisting of propylene glycol, 2-propanol, 1,3-butanediol and ethylene glycol.
(8) The nonaqueous external preparation according to any of the above items (1) to (7) wherein the basic pharmacologically active component is lidocaine and the organic acid is lactic acid.
(9) The nonaqueous external preparation according to any of the above items (1) to (7) wherein the basic pharmacologically active component is morphine and the organic acid is selected from the group consisting of benzoic acid, salicylic acid, acetylsalicylic acid and 3-hydroxybutyric acid.
(10) The nonaqueous external preparation according to any of the above items (1) to (9) wherein the external preparation is a tape preparation.

(11) A levulinic acid salt of a basic pharmacologically active component selected from the group consisting of tramadol, lidocaine, pentazocine, tolperisone, eperisone, amitriptyline, imipramine, donepezil and morphine.
(12) A lactic acid salt of a basic pharmacologically active component selected from the group consisting of tramadol, lidocaine, pentazocine, tolperisone, eperisone, amitriptyline, imipramine, donepezil and morphine.
(13) A method for production of a nonaqueous external preparation comprising a basic pharmacologically active component having an excellent transdermal absorbability which comprises the following steps:
   a) selecting a compound having a logP of 0.5 to 5 as a basic pharmacologically active component;
   b) selecting a compound having a logP of -1 to 4 as an organic acid to form an equimolecular salt thereof;
   c) dissolving the equimolecular salt in an ester solvent and/or an alcohol solvent; and
   d) blending and dispersing the above solution into a base of the external preparation.
(14) The method for production of the external preparation according to the above item (13) wherein the basic pharmacologically active component is one or more selected from the group consisting of tramadol, lidocaine, pentazocine, tolperisone, eperisone, amitriptyline, imipramine, donepezil and morphine.
(15) The method for production of the external preparation according to the above item (13) wherein the basic pharmacologically active component is a compound having the logP of 2 to 5.
(16) The method for production of the external preparation according to any of the above items (13) to (15) wherein the organic acid is selected from the group consisting of acetic acid, propionic acid, butyric acid, hexanoic acid, glycolic acid, methoxyacetic acid, lactic acid, levulinic acid, benzoic acid, salicylic acid, acetylsalicylic acid and 3-hydroxybutyric acid.
(17) The method for production of the external preparation according to any of the above items (13) to (16) wherein the ester solvent is one or more selected from the group consisting of isopropyl myristate, diethyl sebacate and medium-chain fatty acid triglyceride.
(18) The method for production of the external preparation according to any of the above items (13) to (17) wherein the alcohol solvent is one or more selected from the group consisting of isopropanol, ethylene glycol, propylene glycol and 1,3-butanediol.

### EFFECT OF INVENTION

A nonaqueous external preparation of the present invention is an external preparation comprising an organic acid salt as an active component made from a basic pharmacologically active component having a lipid solubility (logP value) of 0.5 to 5 and an organic acid having a lipid solubility (logP value) of -1 to 4. Further, the external preparation of the present invention is prepared by solvating the organic acid salt in a mixed solvent of an ester solvent and an alcohol solvent followed by dissolving and dispersing the solvated organic acid salt into a base of an external preparation. The external preparation having such composition achieves high preservation stability without precipitation of the active component having high concentration of the active component as well as an excellent transdermal absorbability.
According to a method for producing a nonaqueous external preparation of the present invention, an equimolar salt of a basic pharmacologically active component having a lipid solubility (logP value) of 0.5 to 5 and an organic acid having a lipid solubility (logP value) of -1 to 4 is prepared. Then, the organic acid salt is solvated in a mixed solvent of an ester solvent and an alcohol solvent followed by dissolving and dispersing the solvate into a base of an external preparation. As such, by the method of the present, an external preparation having high preservation stability without precipitation of the active component having high concentration of the active component as well as an excellent transdermal absorbability is prepared. That is, the increased lipid solubility (logP) of the organic acid salt makes the salt easy to be dissolved and dispersed uniformly in a circumstance having higher lipid solubility (such as a solvent or a base). However, too high lipid solubility reduces a transdermal absorbability of the salt. As a result of selection of a suitable combination of the solvents, the uniform dispersion of the organic acid salt in a base of an external preparation becomes possible and thereby an external preparation having an excellent transdermal absorbability can be produced.

### BRIEF DESCRIPTION OF DRAWINGS

[Figure 1] Comparison of a transdermal absorbability (skin penetrability) between a liquid preparation of morphine and liquid preparations of an organic acid (carboxylic acid) salt of morphine is depicted.
[Figure 2] Comparison of a transdermal absorbability (skin penetrability) between a liquid preparation of pentazocine and liquid preparations of an organic acid (carboxylic acid) salt of pentazocine is depicted.
[Figure 3] Correlation of "a transdermal absorbability (skin penetrability) of a liquid preparation of pentazocine and liquid preparations of an organic acid (carboxylic acid) salt of pentazocine" with "lipid solubility of carboxylic acid (logP value)" is depicted.
[Figure 4] Comparison of a transdermal absorbability (skin penetrability) between a liquid preparation of amitriptyline and liquid preparations of an organic acid (carboxylic acid) salt of amitriptyline is depicted.
[Figure 5] Correlation of "a transdermal absorbability (skin penetrability) of a liquid preparation of amitriptyline and liquid preparations of an organic acid (carboxylic acid) salt of amitriptyline" with "lipid solubility of carboxylic acid (logP value)" is depicted.
[Figure 6] Comparison of a transdermal absorbability (skin penetrability) between a liquid preparation of tramadol and liquid preparations of an organic acid (carboxylic acid) salt of tramadol is depicted.
[Figure 7] Correlation of "a transdermal absorbability (skin penetrability) of a liquid preparation of tramadol and liquid preparations of an organic acid (carboxylic acid) salt of tramadol" with "lipid solubility of carboxylic acid (logP value)" is depicted.
[Figure 8] Comparison of a transdermal absorbability (skin penetrability) between a liquid preparation of donepezil and liquid preparations of an organic acid (carboxylic acid) salt of donepezil is depicted.
[Figure 9] Correlation of "a transdermal absorbability (skin penetrability) of a liquid preparation of donepezil and liquid preparations of an organic acid (carboxylic acid) salt of donepezil" with "lipid solubility of carboxylic acid (logP value)" is depicted.
[Figure 10] Comparison of a transdermal absorbability (skin penetrability) between a liquid preparation of lidocaine (2 %) and liquid preparations of an organic acid (carboxylic acid) salt of lidocaine (2 %) is depicted.
[Figure 11] Comparison of a transdermal absorbability (skin penetrability) between a liquid preparation of lidocaine (20 %) and liquid preparations of an organic acid (carboxylic acid) salt of lidocaine (20 %) is depicted.
[Figure 12] Comparison of the effects on the tissue permeability due to a difference of solvent using a tape preparation containing 20 % of lidocaine lactic acid salt is depicted.
[Figure 13] Comparison of the effects on the tissue permeability due to a difference of solvent using a tape preparation containing 15 % of lidocaine lactic acid salt is depicted.
[Figure 14] Comparison of the effects on a transdermal absorbability due to change of a combination of non-ionic surfactants (HLB value) using a tape preparation containing 20 % of lidocaine lactic acid salt is depicted.

### BEST MODE FOR CARRYING OUT THE INVENTION

### The first aspect of the present invention

The first aspect of the present invention relates to an external preparation comprising an organic acid salt of a basic pharmacologically active component.
The term "a basic pharmacologically active component" as used herein means a basic compound having a primary, secondary or tertiary amine structure as a functional group and includes local analgesics such as lidocaine, dibucaine, bupivacaine, procaine, mepivacaine, bupivacaine and tetracaine; antihistaminics such as diphenhydramine; analgesics such as tramadol, pentazocine and buprenorphine; muscle relaxants such as eperisone and tolperisone; antitussives such as dextromethorphan; degradation inhibitors of acetylcholine such as donepezil; antidepressants such as imipramine, amitriptyline and paroxetine; calcium antagonists such as amlodipine; and narcotics such as morphine, heroin and codeine.
Preferable basic pharmacologically active components have a logP of 0.5 to 5. More preferable basic pharmacologically active components have a logP of 2 to 5 and include, for example, tramadol, lidocaine, pentazocine, tolperisone, eperisone, amitriptyline, imipramine and donepezil.

The term "organic acid" as used herein is short-chain fatty acids having 2 to 7 carbon atoms such as acetic acid, butyric acid and hexanoic acid; medium-chain fatty acids having 8 to 11 carbon atoms such as octanoic acid and decanoic acid; long-chain fatty acids having more than 12 carbon atoms such as myristic acid, stearic acid and isostearic acid; short-chain fatty acids substituted with a hydroxy group, an alkoxy group or an acyl group such as glycolic acid, lactic acid, methoxyacetic acid, mandelic acid, levulinic acid and 3-hydroxybutyric acid; benzenecarboxylic acid such as benzoic acid, p -hydroxybenzoic acid, salicylic acid and acetylsalicylic acid; and organosulfonic acid such as benzenesulfonic acid, toluenesulfonic acid, menthylsulfonic acid and dodecylbenzenesulfonic acid.
P referable organic acids have a logP of -1 to 4 and include short-chain fatty acids having 2 to 7 carbon atoms such as acetic acid, butyric acid and hexanoic acid; short-chain fatty acids substituted with a hydroxy group, an alkoxy group or an acyl group such as glycolic acid, lactic acid, methoxyacetic acid, 3-hydroxybutyric acid, mandelic acid and levulinic acid; and benzenecarboxylic acid such as benzoic acid, p - hydroxybenzoic acid, salicylic acid and acetylsalicylic acid. More preferable organic acids have a logP of -1 to 2 and include, for example, levulinic acid, lactic acid, acetic acid, benzoic acid, salicylic acid, methoxyacetic acid, hexanoic acid, glycolic acid and 3-hydroxybutyric acid.

The term "external preparation" as used herein means, for example, a liquid preparation, an ointment, a plaster (tape preparation) and a cream. Preferable external preparations include a liquid preparation, an ointment and a plaster (tape preparation).
An external preparation of the present invention essentially contains an ester solvent, an alcohol solvent, and a transdermal absorption promoter, and further may include some additives which are well-known by those skilled in the art and are commonly used depending on a dosage form. Such additives include, for example, a base, a surfactant, a suspending agent, a thickener, inorganic particles, a stabilizer, a buffering agent, a pH adjuster, a coloring agent and a flavor.
The term "nonaqueous" as used herein means that a preparation does not contain water as an essential ingredient. That is, the term "nonaqueous external preparation" as used herein is an external preparation which does not contain water as an essential ingredient, and therefore, moisture which is normally attached to or stored in a substance or a solvent is exempt. For example, among patches, while a poultice which contains water as an essential ingredient does not fall into the nonaqueous external preparation, a plaster (tape preparation) falls into the nonaqueous external preparation.

The term "ester solvent" as used herein means esters of long-chain fatty acids such as oleic acid, capric acid, capronic acid, myristic acid, palmitic acid and stearic acid, and monovalent aliphatic alcohols (for example, myristate esters such as isopropyl myristate and ethyl myristate; palmitate esters such as isopropyl palmitate and ethyl palmitate; stearate esters such as isopropyl stearate; and oleate esters such as decyl oleate are included); medium-chain fatty acid triglyceride such as capric tryglyceride, caproic tryglyceride, peanut oil, olive oil, castor oil, cacao oil and hydrogenated oil (for example, hydrogenated castor oil); esters of polyvalent carboxylic acids such as adipic acid and sebacic acid, and monovalent aliphatic alcohols (for example, sebacate esters such as diethyl sebacate and diisopropyl sebacate; and adipate esters such as diethyl adipate and diisopropyl adipate are included); and carbonate esters such as propylene carbonate.
Preferable ester solvents include myristate esters such as isopropyl myristate and ethyl myristate; medium-chain fatty acid triglycerides such as capric tryglyceride, caproic tryglyceride, peanut oil, olive oil, castor oil, cacao oil and hydrogenated oil (for example, hydrogenated castor oil); sebacate esters such as diethyl sebacate and diisopropyl sebacate; and propylene carbonate. More preferable ester solvents include isopropyl myristate, olive oil, diethyl sebacate and propylene carbonate.

The term "alcohol solvent" as used herein means higher alcohols such as benzyl alcohol, lauryl alcohol, myristyl alcohol, cetyl alcohol, stearyl alcohol, cetearyl alcohol and 2-octyldodecanol; lower alcohols such as ethanol, isopropanol and n-propanol; and polyols such as ethylene glycol, glycerin, propylene glycol and 1,3-butanediol.
Prerferable alcohol solvents include lower alcohols such as ethanol, isopropanol and n-propanol; and polyols such as ethylene glycol, glycerin, propylene glycol and 1,3-butanediol. More preferable alcohol solvents include isopropanol, ethylene glycol, glycerin, propylene glycol and 1,3-butanediol.

The term "transdermal absorption promoter" as used herein means reagents which are commonly used in the field of patches for promoting transdermal absorption of medicaments. The transdermal absorption promoters include, for example, the above listed organic acids, alcohol solvents and ester solvents as well as menthol, n-methylpyrrolidone, limonene, and crotamiton. In addition, the same or different organic acids as those used for producing the organic acid salts of basic pharmacologically active components may be used as a transdermal absorption promoter. For example, in the case of an acetic acid salt or a lactic acid salt of a basic pharmacologically active component, acetic acid or lactic acid may be added as a transdermal absorption promoter and the different organic acids such as oleic acid, levulinic acid, myristic acid, stearic acid or isostearic acid may also used as the promoter. Preferable transdermal absorption promoters include such as menthol, n-methylpyrrolidone as well as low low-volatile fatty acids such as lactic acid, oleic acid and levulinic acid.

As a base of external preparations, for the case of a dosage form of a liquid preparation, commonly-used reagents and generally-used solvents such as the above listed alcohol solvents and ester solvents can be used.
For the case of a dosage form of an ointment, a base includes oils such as vaseline, cetanol, beeswax, bleached wax, lanolin, purified lanolin, liquid paraffin, paraffin wax, plastibase containing liquid paraffin and polyethylene, silicon oil, medium-chain fatty acid triglyceride, squalene, microcrystalline wax and cetaceum.
For the case of a dosage form of a plaster (tape preparation), a base includes rubbers such as natural rubber, isoprene rubber, polyisobutylene, styrene-isoprene-styrene block copolymer, styrenebutadiene-styrene block copolymer, styrene-ethylene-butylene-styrene block copolymer, alkyl (metha) acrylate ester (co)polymer, polyacrylate ester, methacrylate ester, polyisobutylene, polybutene and liquid polyisoprene.

The term "excellent transdermal absorbability" as used herein means that an external preparation of the present invention has a superior transdermal absorbability (skin penetrability) than an external preparation which includes a basic pharmacologically active component in a free form in the absence of an organic acid. Thus, an external preparation having a superior transdermal absorbability (skin penetrability) than an external preparation in the free form of a basic pharmacologically active component falls into the present invention.
Surfactants which can be added to an external preparation of the present invention include "anionic surfactants", for example, natural emulsifiers such as gum arabic, gelatin, tragacanth, lecitin and cholesterol; soap; and sodium alkylsulfate such as sodium lauryl sulfate; "nonionic surfactants", for example, polyoxyethylene sorbitan fatty acid esters such as monooleyl polyoxyethylene sorbitan; glycerin fatty acid esters such as polyoxyethylene castor oil derivatives, polyoxyethylene hydrogenated castor oil, glycerin monostearate and sorbitan monooleate; sorbitan fatty acid esters such as sorbitan monostearate and sorbitan sesquiolate; polyoxyethylene higher alcohol ethers such as polyoxyethylenecetyl ether; polyoxyethylene alkylphenol and polyoxyethyleneoxypropylene copolymer (for example, Pluronic); "cationic surfactants" such as cetyltrimethylammonium chloride; and "amphoteric surfactants".
Depending on properties of an organic acid salt of a basic pharmacologically active component (for example, lipid solubility, state of charge and entire shape), the dispersibility of the organic acid salt into a base of an external preparation can be enhanced by using a suitable surfactant. Preferable surfactants include "anionic surfactants", for example, sodium alkylsulfate such as sodium lauryl sulfate; and "nonionic surfactants" such as polyoxyethylene castor oil derivatives, polyoxyethylene hydrogenated castor oil, glycerin monostearate and sorbitan monooleate. More preferable surfactants include "nonionic surfactants" such as polyoxyethylene castor oil derivatives, polyoxyethylene hydrogenated castor oil, glycerin monostearate and sorbitan monooleate. Further, a combination of these nonionic surfactants can also be used.

Suspending agents or thickeners which can be added to an external preparation of the present invention include polysaccharides such as gum arabic, tragacanth, pullulan, locust bean gum, tamarind gum, pectin, xanthane gum, guar gum and carrageenan; methylcellulose, carmellose, carmellose sodium, polyvinyl alcohol, polyvinylpyrrolidone, acrylate copolymer, carboxy vinyl polymer and colloidal microcrystalline cellulose.

Inorganic particles which can be added to an external preparation of the present invention include, for example, talc, silicic anhydride, calcium carbonate, magnesium carbonate, colloidal silica and bentonite. Texture of the preparation to skin can be controlled by adding these particles.

Stabilizers which can be added to an external preparation of the present invention include, for example, a preservative and an antioxidant. Said preservatives include, for example, parahydroxybenzoate esters such as methylparaben, propylparaben; alcohols such as chlorobutanol, benzyl alcohol and phenylethyl alcohol; thimerosal, anhydrous acetic acid and sorbic acid. Said antioxidants include, for example, sodium bisulfite, L-ascorbic acid, sodium ascorbate, butylhydroxyanisole, butylhydroxytoluene, propyl gallate, tocopheryl acetate and dl-α-tocopherol.

Also, in addition to these additives, a medicinal product containing any other drugs which do not interfere with the functional effects of an external preparation of the present invention can be added to the preparation.
The above exemplified additives can be appropriately selected depending on a dosage form of an external preparation of the present invention. An amount thereof also can appropriately be selected depending on the dosage form within the range of amounts to be used normally.

An external preparation of the present invention can be applied to the affected area depending on the dosage form. An amount of the external preparation to be applied can be selectetd depending on the amount of an active ingredient in the external preparation. For example, the external preparation can be applied one or more times per day and the frequency of application is unlimited.

### The second aspect of the present invention

The second aspect of the present invention relates to a method for production of an external preparation comprising an organic acid salt of a basic pharmacologically active component having an excellent transdermal absorbability.
Any commonly-used known means can be utilized in the method. In particular, the method of the present invention comprising a basic pharmacologically active component is characterized in the following steps. That is, the method for production of an external preparation having an excellent transdermal absorbability comprises the following steps:
1) selecting a compound having a logP of 0.5 to 5 as a basic pharmacologically active component;
2) selecting a compound having a logP of -1 to 4 as an organic acid to form an equimolecular salt thereof;
3) dissolving the equimolecular salt in an ester solvent and/or an alcohol solvent; and
4) blending and dispersing the above solution into a base of the external preparation.

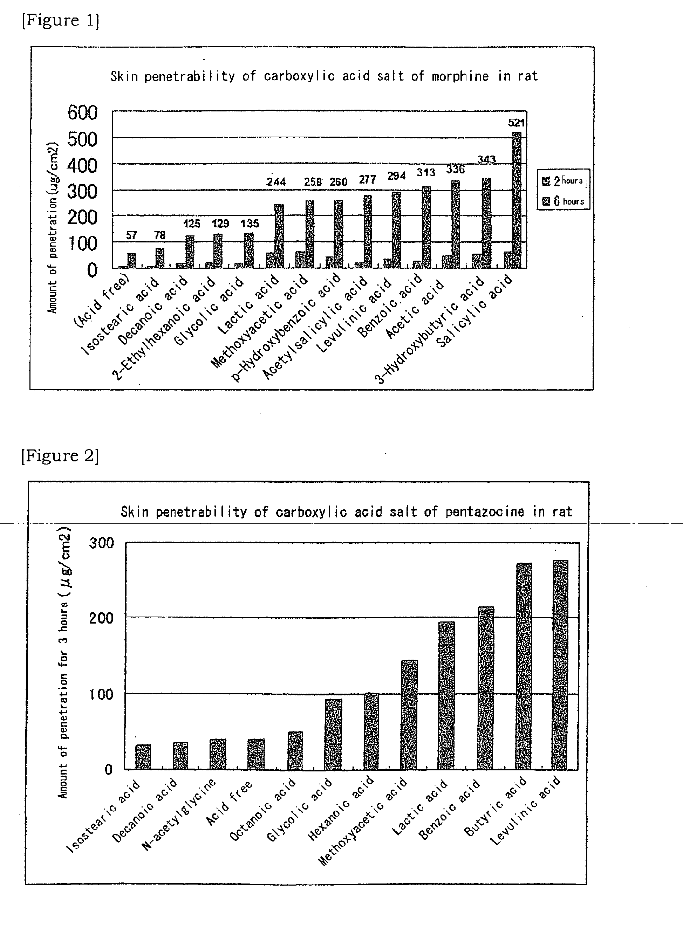

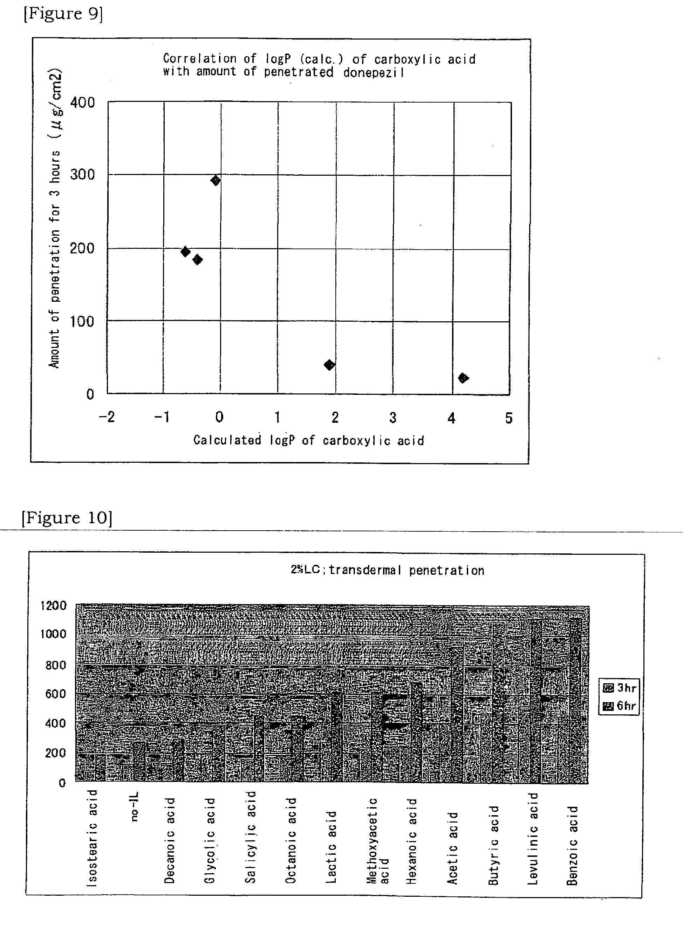

The definitions of the terms used herein and the other additives are the same as those described in the first aspect of the present invention above.
A base of various external preparations has different composition and lipid solubility depending on the dosage form such as a liquid preparation, a cream and a plaster (tape preparation). If each of a basic pharmacologically active component and an organic acid has high lipid solubility, it has a tendency to be dispersed into a base having higher lipid solubility. In contrast, if lipid solubility of an organic acid salt of a basic pharmacologically active component becomes too high, a transdermal absorbability of the salt becomes to be reduced. Therefore, as described above, it is preferable that a salt is formed from a compound having a logP of 0.5 to 5 as a basic pharmacologically active component and a compound having a logP of - 1 to 4 as an organic acid. It is more preferable that a salt is formed from a compound having a logP of 2 to 5 as a basic pharmacologically active component and a compound having a logP of -1 to 2 as an organic acid.
For example, when a liquid preparation was prepared from pentazocine (logP of 3.7) as an basic pharmacologically active component and a variety of organic acids, a transdermal absorbability of the salt had a tendency as shown in Figure 2. On the other hand, when lidocaine (logP of 2.1) was used as a basic pharmacologically active component, a transdermal absorbability of the salt had a tendency as shown in Figure 10. As shown in Table 12, when a liquid preparation is prepared from lidocaine, a fatty acid having higher lipid solubility can be used as an organic acid compared with a preparation prepared from pentazocine.

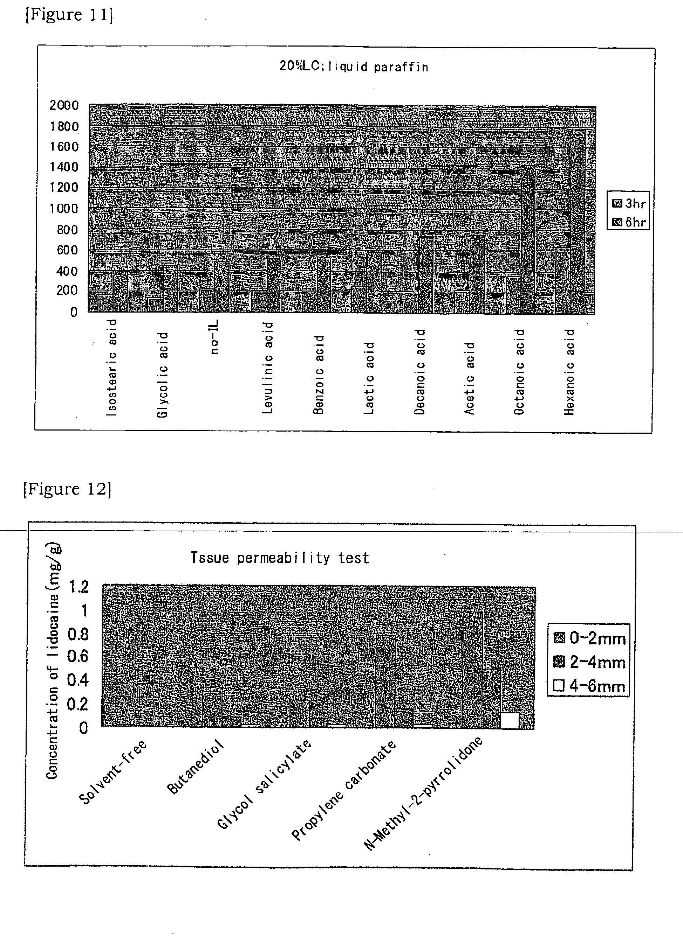

In addition, viscosity of a base of an external preparation also affects a transdermal absorbability. That is, if the viscosity becomes higher, a transdermal absorbability is reduced as a result of suppression of mass transfer of a basic pharmacologically active component in the base. For example, a liquid preparation was compared with a cream preparation as to lidocaine. When both of the viscosity and the lipid solubility are increased, a transdermal absorbability is extremely reduced as shown in Table 17, Figure 10 and Figure 11.
However, the mass transfer can be controlled by selecting a suitable ester solvent and/or alcohol solvent for solvating the organic acid salt. The dispersion of the organic acid salt can be further promoted by adding a surfactant. As a result, the selection of a solvent and a surfactant makes it possible to produce a preparation such as an ointment and a plaster (tape preparation) having a more excellent transdermal absorbability.

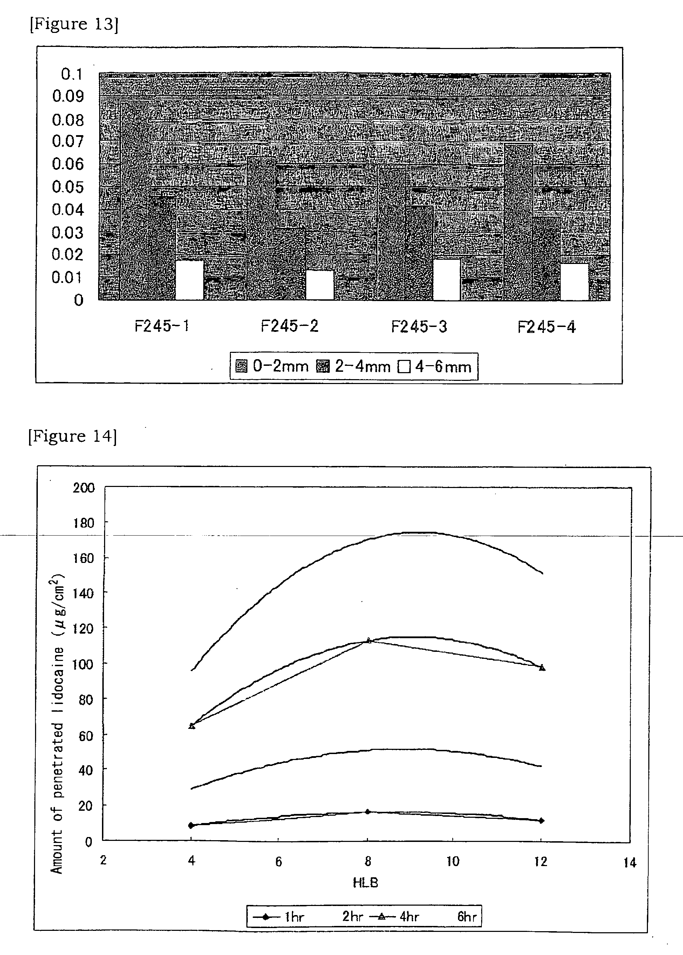

Surfactants which can be used in the present invention include nonionic surfactants such as polyoxyethylene castor oil derivatives, polyoxyethylene hydrogenated castor oil, glycerin monostearate and sorbitan monooleate. A combination of these nonionic surfactants can also be used. If a combination of the nonionic surfactants is used, it is preferable that the combined surfactants have a HLB value of 6 to 12 as shown in Figure 13.

The present invention is illustrated in more details by the following Examples. However, the present invention is not limited to these Examples and may be carried out with any modifications. All of such modifications are within the technical scope of the invention.

### EXAMPLES

### (Example 1) Synthesis of levulinic acid salt of pentazocine

500 mg (1.75 mM) of pentazocine (pKa 8.0) and 225 mg (1.75 mM) of levulinic acid were dissolved in 0.5 mL of tetrahydrofuran, and then, tetrahydrofuran was distilled off in vacuo to obtain a levulinic acid salt of pentazocine as a viscous liquid.
Infrared absorption spectrum (neat, cm⁻¹): 1710 (4-position carbonyl of levulinic acid), 1580 (carbonyl of carboxyl anion)
In the infrared absorption spectrum, a wide absorption band of starting levulinic acid near 1700∼1720 cm⁻¹ changed to a narrow absorption band and an absorption band of carboxyl ion (1580 cm⁻¹) newly appeared.

### (Example 2) Synthesis of an equimolecular salt of a basic pharmacologically active component and levulinic acid

An equimolar amount of a basic pharmacologically active component and levulinic acid were dissolved in tetrahydrofuran to obtain quantitatively the following salts of a basic pharmacologically active component and levulinic acid according to the same procedure as Example 1.

**[Table 1]**

| Sample No. | Basic pharmacologically active component | IR absorption of salt νC=O(cm⁻¹) of carboxy anion | Form of salt |
|---|---|---|---|
| 1 | amitriptyline | 1580 | viscous liquid |
| 2 | imipramine | 1580 | viscous liquid |
| 3 | lidocaine | 1580 | viscous liquid |
| 4 | tramadol | 1580 | viscous liquid |
| 5 | eperisone | 1570 | viscous liquid |
| 6 | tolperisone | 1570 | viscous liquid |
| 7 | donepezil | 1580 | viscous liquid |

It was confirmed from the infrared absorption spectrum in the above Table 1 that an absorption band (1700∼1720 cm⁻¹) of a carboxyl group of the starting levulinic acid disappeared and an absorption band near 1570∼1580 cm⁻¹ newly appeared, and therefore, a production of a levulinic acid salt of the basic pharmacologically active component was confirmed.

### (Example 3) Synthesis of a lactic acid salt of lidocaine

234 mg (1 mM) of lidocaine (pKa 7.9) and 102 mg (content: 88.5%, 1 mM) of lactic acid were dissolved in 1 mL of tetrahydrofuran, and then, tetrahydrofuran was distilled off in vacuo to obtain a lactic acid salt of lidocaine as a viscous liquid.
Infrared absorption spectrum (neat, cm⁻¹): 1590 (carbonyl of carboxyl anion)
In the infrared absorption spectrum, an absorption band of starting lactic acid near 1730 cm⁻¹ disappeared and an absorption band of carboxyl ion (1590 cm⁻¹) newly appeared.

### (Example 4) Synthesis of an equimolecular salt of a basic pharmacologically active component and lactic acid

An equimolar amount of a basic pharmacologically active component and lactic acid were dissolved in tetrahydrofuran to obtain quantitatively the following salts of a basic pharmacologically active component and lactic acid according to the same procedure as Example 3.

**[Table 2]**

| Sample No. | Basic pharmacologically active component | IR absorption of salt νC=O(cm⁻¹) of carboxy anion | Form of salt |
|---|---|---|---|
| 1 | amitriptyline | 1600 (neat) | viscous liquid |
| 2 | imipramine | 1600 (neat) | viscous liquid |
| 3 | pentazocine | 1580 (chloroform) | white powder |
| 4 | tramadol | 1590 (chloroform) | viscous liquid |
| 5 | eperisone | 1590 (neat) | viscous liquid |
| 6 | tolperisone | 1590 (neat) | viscous liquid |
| 7 | donepezil | 1580 (neat) | viscous liquid |

It was confirmed from the infrared absorption spectrum in the above Table 2 that an absorption band (1730 cm⁻¹) of a carboxyl group of the starting lactic acid disappeared and an absorption band near 1570∼1600 cm⁻¹ newly appeared, and therefore, a production of a lactic acid salt of the basic pharmacologically active component was confirmed.

### (Example 5) A liquid preparation containing a levulinic acid salt of pentazocine

200 mg (0.7 mM) of pentazocine is weighed into a sample container and 81 mg (0.7 mM) of levulinic acid is weighed into the container. Then, a solvent having a mass ratio of 45:25:15:6:2:2:5 (isopropyl myristate:medium-chain fatty acid triglyceride:diethyl sebacate:n-methylpyrrolidone:propylene glycol:1-menthol:2-propanol) was added to the container to bring the total amount to 10 g. A liquid preparation of a pentazocine-levulinic acid salt containing 2 w/w% of pentazocine was produced by stirring to homogenize the liquid preparation at room temperature.

### (Example 6) A liquid preparation containing a levulinic acid salt of amitriptyline

200 mg (0.7 mM) of amitriptyline is weighed into a sample container and 84 mg (0.7 mM) of levulinic acid is weighed into the container. Then, a solvent having a mass ratio of 45:25:15:6:2:2:5 (isopropyl myristate:medium-chain fatty acid triglyceride:diethyl sebacate:n-methylpyrrolidone:propylene glycol:1-menthol:2-propanol) was added to the container to bring the total amount to 10 g. A liquid preparation of an amitriptyline-levulinic acid salt containing 2 w/w% of amitriptyline was produced by stirring to homogenize the liquid preparation at room temperature.

### (Example 7) A liquid preparation containing a hexanoic acid salt of tramadol

200 mg (0.76 mM) of tramadol is weighed into a sample container and 88 mg (0.76 mM) of hexanoic acid is weighed into the container. Then, a solvent having a mass ratio of 45:25:15:6:2:2:5 (isopropyl myristate:medium-chain fatty acid triglyceride:diethyl sebacate:n-methylpyrrolidone:propylene glycol:1-menthol:2-propanol) was added to the container to bring the total amount to 10 g. A liquid preparation of an amitriptyline-levulinic acid salt containing 2 w/w% of amitriptyline was produced by stirring to homogenize the liquid preparation at room temperature.

### (Example 8) A liquid preparation containing a lactic acid salt of lidocaine

200 mg (0.85 mM) of lidocaine is weighed into a sample container and 85 mg (90.2%, 0.85 mM) of lactic acid is weighed into the container. Then, a solvent having a mass ratio of 45:25:15:6:2:2:5 (isopropyl myristate:medium-chain fatty acid triglyceride:diethyl sebacate:n-methylpyrrolidone:propylene glycol:1-menthol:2-propanol) was added to the container to bring the total amount to 10 g. A liquid preparation of a lidocaine-lactic acid salt containing 2 w/w% of lidocaine was produced by stirring to homogenize the liquid preparation at room temperature.

### (Example 9) A liquid preparation containing an organic acid salt of morphine

143 mg (0.50 mM) of morphine (pKa 7.9) was weighed into a sample container and an organic acid (0.45 mM) in the following Table 3 was weighed into the container to obtain a homogeneous solution in 0.5 ml of methanol. Methanol was removed by air drying to obtain a crystal or a viscous solution. A fraction of this crystal or viscous solution was isolated and IR absorption spectrum was measured.
Model for IR measurement: FT/IR-6300 type A
Method for measurement: ATR (attenuated total reflection method)

**[Table 3]**

| Organic acid | IR absorption of νC=O(cm⁻¹) | | Form of salt Crystal/Melting point |
|---|---|---|---|
| | organic acid | salt | |
| none | | | crystal : 254-256°C |
| isostearic acid | 1702 | | crystal : 135°C |
| decanoic acid | 1691 | | crystal : 95°C |
| 2-ethylhexanoic acid | 1701 | 1573 | crystal : 145°C, 155°C(two kinds of crystals) |
| salicylic acid | 1656 | 1574¹⁾ | crystal : 179°C, 225°C (two kinds of crystals) |
| benzoic acid | 1679 | 1592 | viscous solution |
| acetylsalicylic acid | 1679 | 1590 | viscous solution |
| p-hydroxybenzoic acid | 1668 | 1594 | viscous solution |
| acetic acid | 1705 | | crystal : over 200°C |
| methoxyacetic acid | 1727 | 1588 | viscous solution |
| lactic acid (90.2%) | 1722 | 1588 | crystal : 132-155°C |
| 3-hydroxybutyric acid | 1707 | 1569¹⁾ | crystal : 115-120°C |
| levulinic acid | 1704 | 1541 | crystal : 67.5°C |
| glycolic acid | 1702 | 1583 | crystal : 98-105°C |

| | | | |
|---|---|---|---|
| [Note] 1) Median value of a broad IR spectrum | | | |

A melting point of free morphine is 254-256 °C. A melting point of an organic acid salt of morphine is lower than that of free morphine. A viscous solution was obtained from a benzoic acid derivative and morphine.

### (Example 10) A liquid preparation containing an organic acid salt of morphine

20 mg (0.07 mM) of morphine (molecular weight 285.4) is weighed into a sample container and an organic acid (0.07 mM) is weighed into the container to obtain compositions (w/w%) listed in the following Table 4. Then, a solvent having a mass ratio of 39:20:8:30 (isopropyl myristate:medium-chain fatty acid triglyceride:n-methylpyrrolidone:2-propanol) was added to the container to bring the total amount to 1000 ng. The liquid preparations of a morphine-organic acid salt containing 2 w/w% of morphine were produced by stirring to homogenize the liquid preparations at room temperature.
Transdermal absorption property was evaluated by using a Franz diffusion cell (penetration area: 2.83 cm², volume of receptor solution: 12 mL) in a rat skin according to Test Example 1.
The results are shown in Table 4 and Figure 1.

**[Table 4]**

| Organic acid (equimolar addition) | logP ¹⁾ | Pentd. Amount ²⁾ (µg/cm²) | Remarks |
|---|---|---|---|
| organic acid-free | | 57 | white precipitation ³⁾ |
| isostearic acid | 7 | 78 | small amount of precipitation ⁴⁾ |
| decanoic acid | 4.2 | 125 | homogeneous solution |
| 2-ethylhexanoic acid | 2.7 | 129 | small amount of precipitation ⁴⁾ |
| salicylic acid | 2.4 | 521 | small amount of precipitation ⁴⁾ |
| benzoic acid | 1.6 | 313 | small amount of precipitation ⁴⁾ |
| acetylsalicylic acid | 1.4 | 277 | homogeneous solution |
| p-hydroxybenzoic acid | 1.2 | 260 | small amount of precipitation ⁴⁾ |
| acetic acid | -0.1 | 336 | small amount of precipitation ⁴⁾ |
| methoxyacetic acid | -0.6 | 258 | homogeneous solution |
| lactic acid (90.2%) | -0.6 | 244 | small amount of precipitation ⁴⁾ |
| 3-hydroxybutyric acid | -0.5 | 343 | small amount of precipitation ⁴⁾ |
| levulinic acid | -0.4 | 294 | homogeneous solution |
| glycolic acid | -1.1 | 135 | homogeneous solution |

| | | | |
|---|---|---|---|
| ¹⁾ Log P of organic acid (Calculated.) ²⁾ Amount of penetrated salt during 6 hours ³⁾ White precipitation of morphine was remained. ⁴⁾ Small amount of white precipitation was remained. [Note] The value from PubMed was used as the calculated logP of an organic acid as with Test Example 1. | | | |

An organic acid salt of morphine has good crystal formability, and therefore, it dissolves insufficiently in production of a liquid preparation. In addition, there is a tendency that equilibrium dissociation of the organic acid salt of morphine and crystallization of a part of the morphine in the solution is caused as a result of a rich content of 2-propanol through the solvation of the salt. However, it has been found that a wide range of organic acids from glycolic acid (logP: - 1.1) to decanoic acid (logP: 4.2) was usable as the organic salt with an excellent transdermal absorbability (skin penetrability).

### (Test Example 1) Evaluation test of a transdermal absorbability of a liquid preparation containing a basic pharmacologically active component

For making a comparative review of 8 kinds of basic pharmacologically active components having a molecular weight of from 245 to 380, according to the solvent composition in Examples 5-8, a liquid preparation containing 2 w/w% of a basic pharmacologically active component was produced by weighing 200 mg of each of basic pharmacologically active components and adding a solvent having a mass ratio of 45:25:15:6:2:2:5 (isopropyl myristate:medium-chain fatty acid triglyceride:diethyl sebacate:n-methylpyrrolidone:propylene glycol:1-menthol:2-propanol) to bring the total amount to 10 g. A fraction of each 100 µL of the above 8 kinds of liquid preparations and the liquid preparation of Example 10 were isolated and an evaluation test of a transdermal absorbability was conducted at 32 °C by using a Franz diffusion cell (penetration area: 1 cm², volume of receptor solution: 8 mL) as follows.
1) Rat skin: an isolated abdominal skin of a five-week old male Wistar rat
2) Receptor solution: saline:ethanol (10:1)
3) Measurement of concentration of penetrated drug: HPLC-ES method (225 nm)
The abdomen of a five-week old Wistar rat was shaved by a clipper on the day before the test. The rat was euthanized with ether, and then, an abdominal skin of the rat was isolated. The skin was set on the vertical diffusion cell (effective diffusion area: 1 cm²), each of the samples listed in Table 5 was applied on the side of a keratinous layer and the saline:ethanol (10:1) solution was applied on the side of a dermic layer. After 3 or 6 hours from the start of the experiment, 100 µL of the saline was sampled, a concentration of the penetrated drug through the skin was measured by HPLC and the accumulated amount of the penetrated-drug was determined.
The amount of the penetrated pharmacologically effective component after 3 hours from the addition of a liquid preparation is shown in Table 5.

**[Table 5]**

| Basic drug | Molecular weight | logP (calc.) | Penetrated Amount ¹⁾ (µg/cm²) |
|---|---|---|---|
| morphine (Example 10) | 285.4 | 0.8 | 57 (during 6 hours) |
| lidocaine | 234.34 | 2.1 | 75 |
| tramadol | 263.38 | 2.4 | 122 |
| tolperisone | 245.36 | 3.1 | 26 |
| donepezil | 379.56 | 3.6 | 25 |
| eperisone | 259.39 | 3.6 | 14 |
| pentazocine | 285.42 | 3.7 | 40 |
| imipramine | 280.41 | 3.9 | 10 |
| amitriptyline | 277.40 | 4.9 | 7 |

| | | | |
|---|---|---|---|
| 1) Amount of skin-penetrated drug during 3 hours [Note] The value published in PubMed which was calculated by a math formula described in "Perspectives in Drug Discovery and Design, Vol. 19, page 47 (2000)" was used as the calculated logP. | | | |

As is clear from the results shown in Table 5, a transdermal absorbability of a basic pharmacologically active component exhibits a bell-shape curve with an increase in the lipid solubility of the pharmacologically active component. That is, it was shown that an excellent transdermal absorbability was exerted by the pharmacologically active component having a lipid solubility within the range of around 2-3 in the case where the component was used alone.
It was also shown that a transdermal absorbability of an organic acid salt of the basic pharmacologically active component generally exhibited a bell-shape curve with an increase of the lipid solubility of the organic acid.

### (Test Example 2) Evaluation test of a transdermal absorbability of an organic acid salt of pentazocine

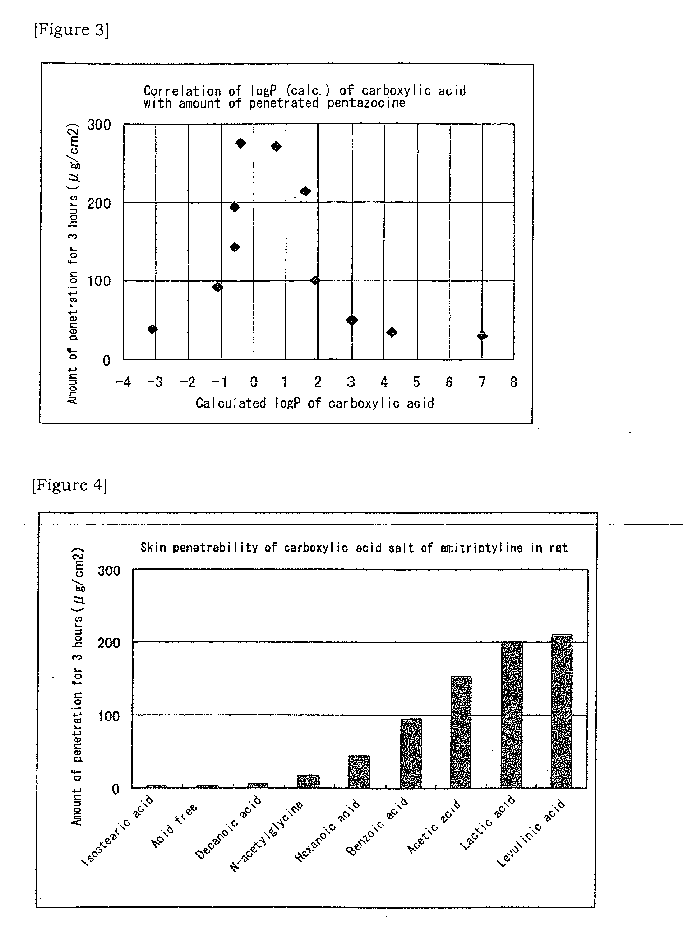

According to the method of Example 5, 20 mg (0.7 mM) of pentazocine is weighed into a sample container (10 mL volume) and an equimolar amount of an organic acid is weighed into the container. Then, a solvent having a mass ratio of 45:25:15:6:2:2:5 (isopropyl myristate:medium-chain fatty acid triglyceride:diethyl sebacate:n-methylpyrrolidone:propylene glycol:1-menthol:2-propanol) was added to the container to bring the total amount to 1000 mg. The liquid preparations of an organic acid salt containing 2 w/w% of pentazocine in Table 6 were produced by stirring to homogenize the liquid preparation at room temperature. A fraction of each 200 µL of the resultant liquid preparations was isolated and a transdermal absorbability was evaluated by using a Franz diffusion cell as described in Test Example 1.
The results were shown in Table 6 below as well as Figures 2 and 3.

**[Table 6]**

| Organic acid (equimolar addition) | Log P of organic acid (calc.) | Penetrated Amount ¹⁾ (µg/cm²) |
|---|---|---|
| organic acid-free | | 40 |
| isostearic acid | 7 | 31 |
| decanoic acid | 4.2 | 35 |
| octanoic acid | 3 | 50 |
| hexanoic acid | 1.9 | 100 |
| benzoic acid | 1.6 | 214 |
| butyric acid | 0.7 | 272 |
| acetic acid | -0.1 | -²⁾ |
| methoxyacetic acid | -0.6 | 143 |
| lactic acid | -0.6 | 194 |
| levulinic acid | -0.4 | 276 |
| glycolic acid | -1.1 | 93 |
| N-acetylglycine | -3.1 | 39 |

| | | |
|---|---|---|
| ¹⁾ Amount of skin-penetrated salt during 3 hours ²⁾ An amount of skin-penetrated salt could not be measured because the acetic acid salt was insoluble in the solvent. [Notes] The value from PubMed was used as the calculated logP of an organic acid as with Test Example 1. | | |

As is clear from these results (Figure 3), it was shown that the organic acid (carboxylic acid) having a logP value of -1 to 2 exhibited a more excellent transdermal absorbability (skin penetrability) than pentazocine alone (organic acid-free).

### (Test Example 3) Evaluation test of a transdermal absorbability of an organic acid salt of amitriptyline

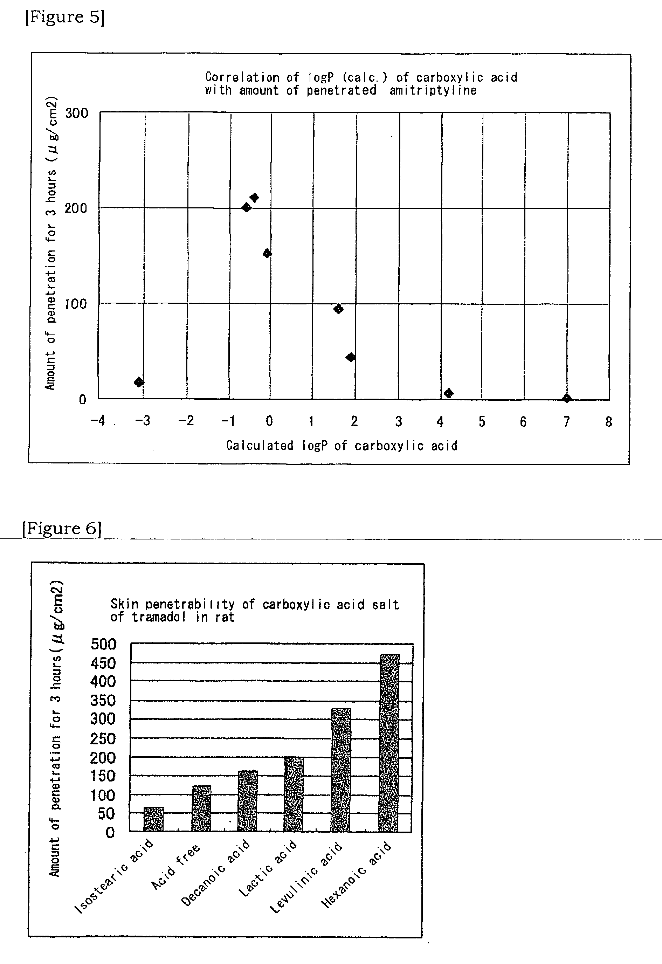

According to the method in Example 6, the liquid preparations (each 1000 mg) of an organic acid salt containing 2 w/w% of amitriptyline in Table 5 below were produced by using 20 mg of amitriptyline. A fraction of each 100 µL of the resultant liquid preparations was isolated and a transdermal absorbability was evaluated by using a Franz diffusion cell as described in Test Example 1.
The results were shown in Table 7 below as well as Figures 4 and 5.

**[Table 7]**

| Organic acid (equimolar addition) | Log P of organic acid (calc.) | Penetrated Amount ¹⁾ (µg/cm²) |
|---|---|---|
| organic acid-free | | 7 |
| isostearic acid | 7 | 2 |
| decanoic acid | 4.2 | 7 |
| hexanoic acid | 1.9 | 44 |
| benzoic acid | 1.6 | 95 |
| acetic acid | -0.1 | 153 |
| levulinic acid | -0.4 | 211 |
| lactic acid | -0.6 | 201 |
| N-acetylglycine | -3.1 | 18 |

| | | |
|---|---|---|
| ¹⁾ Amount of penetrated salt during 3 hours [Note] The value from PubMed was used as the calculated logP of an organic acid as with Test Example 1. | | |

These results showed that, as is clear from Figure 5, the organic acid (carboxylic acid) having a logP value of -1 to 2 exhibited a more excellent transdermal absorbability (skin penetrability) than amitriptyline alone (organic acid-free).

### (Test Example 4) Evaluation test of a transdermal absorbability of an organic acid salt of tramadol

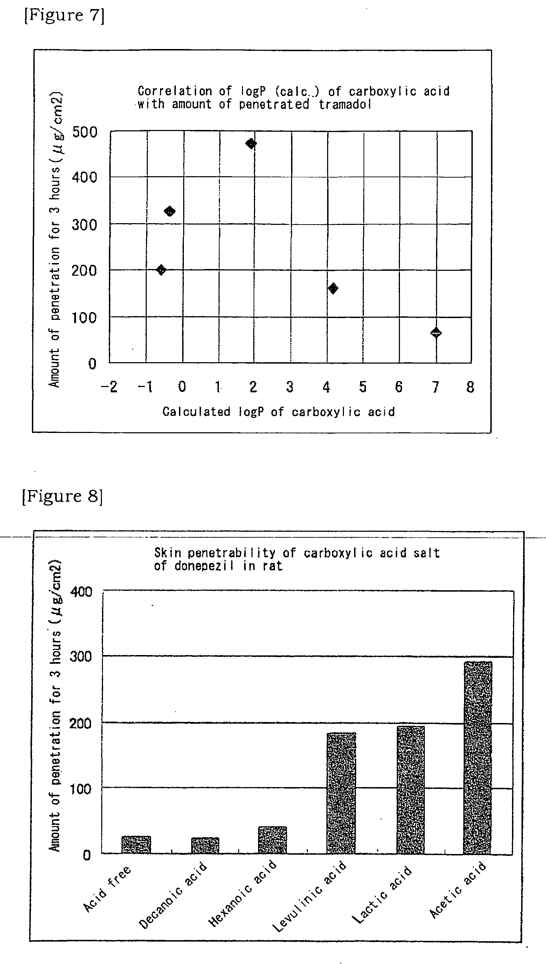

According to the method in Example 7, the liquid preparations (each 1000 mg) of an organic acid salt containing 2 w/w% of tramadol in Table 8 below were produced by using 20 mg of tramadol. A fraction of each 100 µL of the resultant liquid preparations was isolated and a transdermal absorbability was evaluated by using a Franz diffusion cell as described in Test Example 1.
The results were shown in Table 8 below as well as Figures 6 and 7.

**[Table 8]**

| Organic acid (equimolar addition) | logP of organic acid (calc.) | Penetrated Amount ¹⁾ (µg/cm²) |
|---|---|---|
| organic acid-free | | 122 |
| isostearic acid | 7 | 66 |
| decanoic acid | 4.2 | 162 |
| hexanoic acid | 1.9 | 572 |
| levulinic acid | -0.4 | 227 |
| lactic acid | -0.6 | 200 |

| | | |
|---|---|---|
| 1) Amount of penetrated salt during 3 hours [Note] The value from PubMed was used as the calculated logP of an organic acid as with Test Example 1. | | |

These results showed that, as is clear from Figure 7, the organic acid (carboxylic acid) having a logP value of -1 to 4 exhibited a more excellent transdermal absorbability (skin penetrability) than tramadol alone (organic acid-free).

### (Test Example 5) Evaluation test of a transdermal absorbability of an organic acid salt of donepezil

According to the method in Test Example 2, liquid preparations (each 1000 mg) of an organic acid salt containing 2 w/w% of donepezil in Table 9 below were produced by using 20 mg of donepezil. A fraction of each 100 µL of the resultant liquid preparations was isolated and a transdermal absorbability was evaluated by using a Franz diffusion cell as described in Test Example 1.

The results were shown in Table 9 below as well as Figures 8 and 9.

**[Table 9]**

| Organic acid (equimolar addition) | Log P of organic acid (calc.) | Penetrated Amount ¹⁾ (µg/cm²) |
|---|---|---|
| organic acid-free | | 25 |
| decanoic acid | 4.2 | 33 |
| hexanoic acid | 1.9 | 40 |
| acetic acid | -0.1 | 292 |
| lactic acid | -0.6 | 194 |
| levulinic acid | -0.4 | 183 |

| | | |
|---|---|---|
| 1) Amount of penetrated salt for 3 hours [Note] The value from PubMed was used as the calculated logP of an organic acid as with Test Example 1. | | |

These results showed that, as is clear from Figure 9, the organic acid (carboxylic acid) having a logP value of -1 to 2 exhibited a more excellent transdermal absorbability (skin penetrability) than donepezil alone (organic acid-free).

### (Test Example 6) Evaluation test of a transdermal absorbability of an organic acid salt of imipramine

According to the method in Test Example 2, the liquid preparations (each 1000 mg) of an organic acid salt containing 2 w/w% of imipramine in Table 10 below were produced by using 20 mg of imipramine. A fraction of each 100 µL of the resultant liquid preparations was isolated and a transdermal absorbability was evaluated by using a Franz diffusion cell as described in Test Example 1.
The results were shown in Table 10 below.

**[Table 10]**

| Organic acid (equimolar addition) | logP of organic acid (calc.) | Penetrated Amount ¹⁾ (µg/cm²) |
|---|---|---|
| organic acid-free | | 10 |
| acetic acid | -0.1 | 176 |
| lactic acid | -0.6 | 197 |
| levulinic acid | -0.4 | 212 |

| | | |
|---|---|---|
| 1) Amount of penetrated salt during 3 hours [Note] The value from PubMed was used as the calculated logP of an organic acid, as with the above. | | |

These results showed that the organic acid (carboxylic acid) having a logP value of around -1 to 0 exhibited highly a more excellent transdermal absorbability (skin penetrability) than imipramine alone (organic acid-free).

### (Test Example 7) Evaluation test of a transdermal absorbability of an organic acid salt of lidocaine

According to the method in Example 8, the liquid preparations (each 1000 mg) of an organic acid salt containing 2 w/w% of lidocaine in Table 9 below were produced by using 200 mg of lidocaine. A fraction of each 100 µL of the resultant liquid preparations was isolated and a transdermal absorbability was evaluated by using a Franz diffusion cell as described in Test Example 1.
The results were shown in Table 11 below and Figure 10.

**[Table 11]**

| Organic acid (equimolar addition) | logP of organic acid (calc.) | Penetrated Amount ¹⁾ (µg/cm²) |
|---|---|---|
| organic acid-free | | 271 |
| isostearic acid | 7 | 180 |
| decanoic acid | 4.2 | 290 |
| octanoic acid | 3 | 445 |
| hexanoic acid | 1.9 | 675 |
| benzoic acid | 1.6 | 1125 |
| butyric acid | 0.7 | 1090 |
| levulinic acid | -0.4 | 1109 |
| acetic acid | -0.1 | 918 |
| methoxyacetic acid | -0.6 | 614 |
| lactic acid | -0.6 | 608 |
| glycolic acid | -1.1 | 384 |

| | | |
|---|---|---|
| ¹⁾ Amount of penetrated salt during 6 hours [Note] The value from PubMed was used as the calculated logP of an organic acid, as with the above. | | |

These results showed that the organic acid (carboxylic acid) having a logP value of around -1 to 4 exhibited a highly more excellent transdermal absorbability (skin penetrability) than lidocaine alone (organic acid-free).

The above results are tabulated in Table 12 below. A transdermal absorbability of each basic pharmacologically active component exhibits an individual bell-shape curve with an increase in the lipid solubility of an organic acid. There was a tendency that if the lipid solubility of a basic pharmacologically active component was high, the transdermal absorbability became maximal within the range of the organic acids having high hydrophilicity; and if the lipid solubility of a basic pharmacologically active component was low, the transdermal absorbability became maximal within the range of the organic acids having high hydrophobicity. In comparison between amitriptyline (logP: 4.9) and tramadol (logP: 2.4), the transdermal absorbability of a compound having high lipid solubility such as amitriptyline became maximal if the organic acids having the range of a lipid solubility of -0.6 to -0.4 are used. On the other hand, the transdermal absorbability of a compound having low lipid solubility such as tramadol became maximal if the organic acids having a lipid solubility of around 1.9 are used.

**[Table 12]**

| Organic acid (equimolar addition) | logP of organic acid (calc.) | Penetrated Amount during 6 hours (µg/cm²) | | | | |
|---|---|---|---|---|---|---|
| | | AMT | PTZ | TRM | LDC | MRP |
| organic acid-free | | 30 | 169 | 304 | 271 | 57 |
| isostearic acid | 7 | 12 | 111 | 182 | 180 | 78 |
| decanoic acid | 4.2 | 34 | 132 | 423 | 290 | 125 |
| octanoic acid | 3 | | 162 | | 445 | |
| hexanoic acid | 1.9 | | 364 | 1152 | 675 | |
| benzoic acid | 1.6 | 357 | 776 | | 1125 | 313 |
| butyric acid | 0.7 | | 657 | | 1090 | |
| levulinic acid | -0.4 | 818 | 766 | 682 | 1109 | 294 |
| acetic acid | -0.1 | 517 | | | 918 | 336 |
| methoxyacetic acid | -0.6 | | 408 | | 614 | 258 |
| lactic acid | -0.6 | 717 | 574 | 559 | 608 | 244 |
| glycolic acid | -1.1 | | 294 | | 384 | 135 |
| N-acetylglycine | -3.1 | | 247 | | | |

| | | | | | | |
|---|---|---|---|---|---|---|
| AMT: Amitriptyline (logP:4.9) PTZ: Pentazocine (logP:3.7) TRM: Tramadol (logP:2.4) LDC: Lidocaine (logP:2.1) MRP: Morphine (logP:0.8) | | | | | | |

The results of Table 12 showed that, in order to improve the transdermal absorbability of a basic pharmacologically active component, an organic acid (carboxylic acid) should be selected as follows.
a) If a basic pharmacologically active component is a compound having a low lipid solubility such as morphine (logP: 0.8), it is preferable that an organic acid salt of the basic pharmacologically active component is formed using an organic acid having the range of a lipid solubility (logP value) of -1.1 to 4.2. As shown in Examples 10, an organic acid having the range of a logP value of -0.5 to 2.4 was more preferable.
b) If a basic pharmacologically active component is a compound having a relatively-low lipid solubility such as lidocaine (logP: 2.1) and tramadol (logP: 2.4), it is preferable that an organic acid salt of the basic pharmacologically active component is formed using an organic acid having the range of a lipid solubility (logP value) of -1 to 4.2. More preferable organic acids include the organic acids having a logP value of -0.6 to 3.
c) If a basic pharmacologically active component is a compound having a moderate lipid solubility such as pentazocine (logP: 3.7), it is preferable that an organic acid salt of the basic pharmacologically active component is formed using an organic acid having the range of a lipid solubility (logP value) of -1 to 3.0.
d) If a basic pharmacologically active component is a compound having a high lipid solubility such as amitriptyline (logP: 4.9), it is preferable that an organic acid salt of the basic pharmacologically active component is formed using an organic acid having the range of a lipid solubility (logP value) of -1 to 2.

### (Example 11) A liquid preparation containing an equimolar organic acid salt of two kinds of basic pharmacological agents (lidocaine and amitriptyline coexist at a ration of 1:1)

Each 20 mg of lidocaine and amitriptyline is weighed into a 10 mL sample container and an equimolar organic acid is weighed into the container. Then, a solvent having a mass ratio of 45:25:15:6:2:2:5 (isopropyl myristate:medium-chain fatty acid triglyceride:diethyl sebacate:n-methylpyrrolidone:propylene glycol:1-menthol:2-propanol) was added to the container to dissolve the agents for bringing a total amount to 920 mg. Then, 103 µmL (80 mg) of isopropanol was added to the mixture to bring the total amount to 1000 mg. A liquid preparation containing each 2 w/w% of lidocaine and amitriptyline was produced by exposing the liquid preparation to ultrasonic waves for homogenization at room temperature. A fraction of 100 µL of the resultant liquid preparation was isolated and a transdermal absorbability was evaluated by using a Franz diffusion cell as described in Test Example 1.
The results were shown in Table 13 (lidocaine) and Table 14 (amitriptyline) below.

**[Table 13]**

| Organic acid (equimolar addition) | logP of organic acid (calc.) | Penetrated Amount ¹⁾ (µg/cm²) | |
|---|---|---|---|
| | | Coexistence (Example 11) | Alone (Test Example 7) |
| organic acid-free | | 204 | 271 |
| isostearic acid | 7 | | 180 |
| decanoic acid | 4.2 | 310 | 290 |
| salicylic acid | 2.4 | 1240 | 1125 (benzoic acid) |
| propionic acid | 0.2 | 1227 | 1109 (butyric acid) |
| levulinic acid | -0.4 | 1169 | 1109 |
| acetic acid | -0.1 | 1170 | 918 |
| lactic acid | -0.6 | 1047 | 608 |
| glycolic acid | -1.1 | 1037 | 384 |

| | | | |
|---|---|---|---|
| ¹⁾ Amount of penetrated lidocaine during 6 hours [Note] The value from PubMed was used as the calculated logP of an organic acid as with Test Example 1. | | | |

It was shown in the above Table 13 that there was no great difference of a transdermal absorbability between a liquid preparation containing a single basic pharmacologically active component and multiple basic pharmacologically active components. That is, the liquid preparation containing multiple basic pharmacologically active components has the similar transdermal absorbability to those containing a single basic pharmacologically active component. For example, it was shown that a liquid preparation in which lidocaine and amitriptyline coexist (this Example) had the similar transdermal absorbability to those containing lidocaine alone (Test Example 7).

On the other hand, it was shown in the following Table 14 that there was no great difference of a transdermal absorbability between a liquid preparation in which amitriptyline and lidocaine coexist and a liquid preparation containing amitriptyline alone (Test Example 3).

**[Table 14]**

| Organic acid (equimolar addition) | logP of organic acid (calc.) | Penetrated Amount ¹⁾ (µg/cm²) | |
|---|---|---|---|
| | | Coexistence (Example 11) | Alone (Test Example 3) |
| organic acid-free | | 0.3 | 7 |
| isostearic acid | 7 | >0.1 | 2 |
| decanoic acid | 4.2 | 3 | 7 |
| salicylic acid | 2.4 | 28 | 44 |
| acetic acid | -0.1 | 249 | 95 |
| levulinic acid | -0.4 | 88 | 153 |
| lactic acid | -0.6 | 146 | 211 |
| glycolic acid | -1.1 | 290 | 201 |

| | | | |
|---|---|---|---|
| 1) Amount of penetrated amitriptyline during 3 hours [Note] The value from PubMed was used as the calculated logP of an organic acid as with Test Example 1. | | | |

As shown from the results of the above Tables 13 and 14, a lipid solubility of lidocaine and amitriptyline is a logP of 2.1 and 4.9, respectively. Therefore, the range of a logP of an organic acid suitable for each transdermal absorbability is different.
In the case of lidocaine alone, a suitable organic acid having the lower limit of the lipid solubility (logP) is lactic acid (logP: -0.6). However, in the case where amitriptyline coexists with lidocaine, if glycolic acid (logP: -1.1) is used as an organic acid, a transdermal absorbability of the liquid preparation is reduced slightly. Accordingly, the lower limit of lipid solubility of a usable organic acid can be reduced. On the other hand, it was shown in the case of amitriptyline that both of a liquid preparation containing amitriptyline alone and those containing amitriptyline and lidocaine showed an improved transdermal absorbability even if an organic acid having high hydrophilicity such as glycolic acid (logP: -1.1) was used.
In the case of lidocaine, the upper limit of lipid solubility (logP) of a suitable organic acid which provides an excellent transdermal absorbability is below 4.2. However, if the lipid solubility of an organic acid is higher than 4.2, the organic acid provides a poor transdermal absorbability. On the other hand, in the case of amitriptyline, an organic acid having a lipid solubility of below 2.4 provides an excellent transdermal absorbability. If an organic acid has higher lipid solubility than 2.4, it provides a less favorable transdermal absorbability. Thus, it was shown that lidocaine which has lower lipid solubility than amitriptyline could utilize an organic acid having higher lipid solubility.

### (Example 12) A liquid preparation containing an equimolar organic acid salt of two kinds of basic pharmacological agents (lidocaine and amitriptyline coexist at a ration of 1:2)

Lidocaine 20 mg and amitriptyline 40 mg are weighed into a 10 mL sample container and an equimolar organic acid is weighed into the container. Then, a solvent having a mass ratio of 45:25:15:6:2:2:5 (isopropyl myristate:medium-chain fatty acid triglyceride:diethyl sebacate:n-methylpyrrolidone:propylene glycol:1-menthol:2-propanol) is added to the container to dissolve the agents for bringing a total amount to 920 mg. Then, 103 µmL (80 mg) of isopropanol was added to the mixture to bring the total amount to 1000 mg. A liquid preparation containing 2 w/w% of lidocaine and 4 w/w% of amitriptyline was produced by exposing the liquid preparation to ultrasonic waves for homogenization at room temperature. A fraction of 100 µL of the resultant liquid preparation was isolated and a transdermal absorbability was evaluated by using a Franz diffusion cell as described in Test Example 1.
The results were shown in Table 15 (lidocaine) and Table 16 (amitriptyline) below.

**[Table 15]**

| Organic acid (equimolar addition) | Log P of organic acid (calc.) | Penetrated Amount ¹⁾ (µg/cm²) | |
|---|---|---|---|
| | | Coexistence (Example 12) | Coexistence (Example 11) |
| organic acid-free | | 147 | 204 |
| decanoic acid | 4.2 | | 310 |
| salicylic acid | 2.4 | | 1240 |
| propionic acid | 0.2 | | 1227 |
| levulinic acid | -0.4 | 835 | 1169 |
| acetic acid | -0.1 | 1304 | 1170 |
| lactic acid | -0.6 | 1135 | 1047 |
| glycolic acid | -1.1 | | 1037 |

| | | | |
|---|---|---|---|
| 1) Amount of penetrated lidocaine during 6 hours [Note] The value from PubMed was used as the calculated logP of an organic acid as with Test Example 1. | | | |

**[Table 16]**

| Organic acid (equimolar addition) | Log P of organic acid (calc.) | Penetrated Amount ¹⁾ (µg/cm²) | |
|---|---|---|---|
| | | 4% (Example 12) | 2% (Example 11) |
| organic acid-free | | 2.3 | 0.3 |
| isostearic acid | 7 | | <0.1 |
| decanoic acid | 4.2 | | 3 |
| salicylic acid | 2.4 | | 28 |
| acetic acid | -0.1 | 764 | 249 |
| levulinic acid | -0.4 | 257 | 88 |
| lactic acid | -0.6 | 104 | 146 |
| glycolic acid | -1.1 | 200^{*)} | 290 |

| | | | |
|---|---|---|---|
| ¹⁾ Amount of penetrated amitriptyline during 3 hours [Note] The value from PubMed was used as the calculated logP of an organic acid as with Test Example 1. *) A solvent was added to 20 mg of lidocaine and 40 mg of amitriptyline to bring the amount to 900 mg, and then, 100 mg of isopropanol was further added to bring the total amount to 1000 mg. This solution was used for the skin penetrability test in rats. | | | |

The results of the above Table 15 showed that a transdermal absorbability of lidocaine was not highly affected by an increase of coexistent amitriptyline content. In addition, there was no apparent shift of a transdermal absorbability due to a difference in organic acids.
The results of the above Table 16 showed that there was a tendency that if the amitriptyline content became higher, the transdermal absorption properties of a levulinic acid salt and an acetic acid salt increased due to correlation with the content of amitriptyline. However, such correlation was not observed in a hydrophilic organic acid such as lactic acid and glycolic acid.
As described above, even in the case of multiple basic pharmacologically active components, the similar transdermal absorbability to the case of a single basic pharmacologically active component was obtained. Therefore, it was shown that a combination drug containing a regional anesthetic such as lidocaine and an antidepressant such as mitriptyline could be considered as a combination of the single pharmaceutical formulations.

### (Example 13) A viscous liquid preparation containing an organic acid salt of lidocaine

Into a sample container, 2.00 g of lidocaine is weighed, and an equimolar of each of the organic acids listed in the following Table 17 is weighed and added into the container. After stirring and homogenizing, liquid paraffin was added to the mixture to produce a viscous liquid preparation having the total amount of 10.00 g. A fraction of 200 µL of the 20 % lidocaine-containing viscous liquid preparation was isolated and a transdermal absorbability was evaluated by using the Franz diffusion cell as described in Test Example 1.
The results were shown in Table 17 below and compared with the results of Test Example 7.

**[Table 17]**

| Organic acid (equimolar addition) | Log P of organic acid (calc.) | Penetrated Amount ¹⁾ (µg/cm²) | |
|---|---|---|---|
| | | 20% prepn. ²⁾ | 2% prepn. ³⁾ (Test Example 7) |
| organic acid-free | | 484 | 271 |
| isostearic acid | 7 | 398 | 180 |
| decanoic acid | 4.2 | 741 | 290 |
| octanoic acid | 3 | 1432 | 445 |
| hexanoic acid | 1.9 | 1761 | 675 |
| benzoic acid | 1.6 | 525 | 1125 |
| butyric acid | 0.7 | | 1090 |
| levulinic acid | -0.4 | 506 | 1109 |
| acetic acid | -0.1 | 750 | 918 |
| methoxyacetic acid | -0.6 | | 614 |
| lactic acid | -0.6 | 585 | 608 |
| glycolic acid | -1.1 | 442 | 384 |

| | | | |
|---|---|---|---|
| ¹⁾ Amount of penetrated lidocaine during 6 hours ²⁾ 20% viscous liquid preparation ³⁾ 2% liquid preparation (Test Example 7) [Note] The value from PubMed was used as the calculated logP of an organic acid as with Test Example 1. The solutions of from an isostearic acid salt to a hexanoic acid salt became homogeneously-dispersed clear solutions. However, the organic acid salts of from benzoic acid to glycolic acid were difficult to disperse homogeneously, and therefore, the organic salt (ionic liquid) had a tendency to become two-layer after standing. | | | |

As shown from the results of Table 17, a suitable organic acid for making a salt having an excellent transdermal absorbability is the organic acid having the range of a lipid solubility (logP) of -0.6 to 4.2 under the condition that an organic acid salt of lidocaine is homogeneously diffused in an viscous base (liquid paraffin). In particular, it is shown that an organic acid having the range of a lipid solubility of -0.1 to 4.2 is preferable for obtaining a transdermal absorbability of more than 750 µg/cm².
On the other hand, it is shown from the results of Test Example 7 that an organic acid having the range of a lipid solubility of -0.6 to 1.9 is preferable for obtaining a transdermal absorbability of more than 600 µg/cm²_{.}
As is shown above, a transdermal absorbability of an organic acid of lidocaine is susceptible to the surrounding circumstance. That is, there was a tendency that if the lipid solubility of a base, in which an organic acid salt of lidocaine existed, became higher, an organic acid having higher lipid solubility could be used for obtaining an organic acid salt having an excellent transdermal absorbability. In addition, if the lipid solubility of an organic acid is more than 1.9, the organic acid salt of lidocaine is homogeneously blended in a base having high lipid solubility such as liquid paraffin. However, there was a tendency that if the lipid solubility of an organic acid is less than 1.6, the organic salt had a tendency to become two-layer without being blended homogeneously. Accordingly, in the case that a logP of an organic acid is less than 1.6, there are large variations in the transdermal absorption properties.
It is shown from the results of an organic acid salt of lidocaine that the solubility or the dispersibility of an organic acid salt of a basic pharmaceutical agent other than lidocaine can be improved by devising a solvent system in which the organic acid salt is dispersed or dissolved to solvate the organic acid salt. A transdermal absorbability can further be improved by adjusting the lipid solubility of a base in which an organic acid salt exists. For example, the surrounding circumstance of an organic acid salt (the dispersibility in a base; and the lipid solubility of a base) can be adjusted by appropriately combining a proton donor such as an alcohol solvent and a proton acceptor such as an ester solvent, and by selecting a ratio of the solvents to be added.

### (Example 14) Effects of solvent addition on a viscous liquid preparation of lidocaine

Into a sample container, 2.00 g of lidocaine is weighed, and an equimolar of lactic acid (0.86 g) (content of 90.2 %) is weighed into the container. To the mixture, 2.0 g of a solvent having a mass ratio of 1:1:0.32:0.18 (propylene carbonate: 1,3-butanediol:polyoxyethylene hydrogenated castor oil:glycerin monostearate) was added and the mixture is stirred. Then, 7.04 g of liquid paraffin was added to prepare an ointment formulation having the total amount of 10.00 g. A fraction of about 200 µl of the 20 % lidocaine-containing viscous liquid preparation was isolated and a transdermal absorbability was evaluated by using a Franz diffusion cell as described in Test Example 1.
The results were shown in Table 18 below and compared with the results of Test Example 7 and Example 13.

**[Table 18]**

| Organic acid (equimolar addition) | logP of organic acid (calc.) | Penetrated Amount ¹⁾ (µg/cm²) | | |
|---|---|---|---|---|
| | | 20% prep. ²⁾ | 20% prep. ³⁾ (Example 13) | 2% prep. ⁴⁾ (T.Example 7) |
| organic acid-free | | 553 | 484 | 271 |
| isostearic acid | 7 | 329 | 398 | 180 |
| decanoic acid | 4.2 | 508 | 741 | 290 |
| octanoic acid | 3 | 1668 | 1432 | 445 |
| hexanoic acid | 1.9 | | 1761 | 675 |
| benzoic acid | 1.6 | 580 | 525 | 1125 |
| butyric acid | 0.7 | | | 1090 |
| levulinic acid | -0.4 | 769 | 506 | 1109 |
| acetic acid | -0.1 | 734 | 750 | 918 |
| methoxyacetic acid | -0.6 | 226 | | 614 |
| lactic acid | -0.6 | 691 | 585 | 608 |
| glycolic acid | -1.1 | 562 | 442 | 384 |

| | | | | |
|---|---|---|---|---|
| ¹⁾ Amount of penetrated lidocaine during 6 hours ²⁾ 20% viscous liquid preparation (addition of solvent) ³⁾ 20% viscous liquid preparation(Example 13) ⁴⁾ 2% liquid preparation(Test Example 7) | | | | |

Mass transfer of lidocaine in a base is suppressed in a viscous liquid preparation because the viscosity of the base and the lipid solubility in the preparation are higher that those in a liquid preparation of Test Example 7. In addition, it is considered that a lidocaine salt is dispersed in the base instead of being dissolved because the surrounding circumstance (base) of an organic salt of lidocaine has high lipid solubility. There is a tendency that a transdermal absorbability is elevated overall by addition of a solvent. However, as shown in Table 18, it is considered that a transdermal absorbability of a viscous liquid preparation is around one-tenth of a liquid preparation because mass transfer in a base is suppressed due to the viscosity of the base.
On the other hand, a volatile substance or a substance having distinctive smell cannot be used as a solvent or an organic acid for preparing a tape preparation in practice. Also, some solvents and organic acids cause redness or inflammation due to their strong skin irritancy. Therefore, there is a limit to the range of solvents and organic acids which can be used.
So, it seems that levulinic acid and lactic acid could be selected from the organic acids in Table 18 as a low-volatile and low-irritative organic acid. In particular, as shown in Table 19 below, it is considered that lactic acid is insusceptible to a base.

**[Table 19]**

| | Lidocaine | Lactic acid salt of lidocaine | Remarks |
|---|---|---|---|
| Test Example 7: 2% liquid preparation (solvent system) | 271 | 608 | homogeneously dispersed solution |
| Example 14: 20% viscous liquid preparation (solvent+liquid paraffin) | 553 | 691 | tendency of salt to be divided into two layers |
| Example 13: 20% viscous liquid preparation (liquid paraffin) | 484 | 585 | tendency of salt to be divided into two layers |

As shown in Table 18, a levulinic acid salt is susceptible to the surrounding circumstance (base), and therefore, its transdermal absorbability varies largely. However, as shown in Table 19, the transdermal absorbability of a lactic acid salt does not vary largely. Accordingly, a lactic acid salt is suitable for making a practical external preparation of lidocaine.

### (Example 15) A tape preparation containing lidocaine-lactic acid salt (1) Effect of a solvent on 20 % lidocaine-lactic acid salt

### a) Effect on a transdermal absorbability

SIS and a terpene resin were weighed according to the composition (w/w%) in Table 20 below and dissolved in toluene, and then, the solution was stirred on heating after adding polybutene, BHT, MGS, liquid paraffin and solvent. Lidocaine and lactic acid (content of 90.2%) were added to obtain a homogeneous plaster base. The resultant plaster base was applied to a tape and toluene was evaporated to prepare a tape preparation was prepared.
Transdermal absorption property of the tape preparation was measured by using a Franz cell as described in Test Example 1 and the transdermal absorbability (µg /cm²) after 6 hours from the starting point of the test was evaluated. These results were also described in Table 20.

**[Table 20]**

| Test preparation | D395 | C404 | C405 | C406 | C407 |
|---|---|---|---|---|---|
| lidocaine | 20.0 | 20.0 | 20.0 | 20.0 | 20.0 |
| lactic acid (90.2%) | 10.4 | 10.4 | 10.4 | 10.4 | 10.4 |
| surfactant: | | | | | |
| glycerin | 4.0 | 4.0 | 4.0 | 4.0 | 4.0 |
| monostearate | | | | | |
| solvent: | -- | 1,3-butanediol 4.0 | glycol salicylate 4.0 | propylene carbonate 4.0 | n-methyl-pyrrolidone 4.0 |
| softener: liquid paraffin | 13.6 | 9.6 | 9.6 | 9.6 | 9.6 |
| tackifier: | | | | | |
| polybutene | 1.0 | 1.0 | 1.0 | 1.0 | 1.0 |
| terpene resin | 36.0 | 36.0 | 36.0 | 36.0 | 36.0 |
| elastomer: SIS | 14.0 | 14.0 | 14.0 | 14.0 | 14.0 |
| antioxidant: | | | | | |
| BHT | 1.0 | 1.0 | 1.0 | 1.0 | 1.0 |
| precipitation of crystal | none | none | none | none | none |
| transdermal absorbability (µg/cm²) | 95 | 194 | 176 | 463 | 166 |

As shown in the results of the above Table 20, propylene carbonate demonstrates an excellent effect as a solvent for the addition to a base. Further, it was shown that 1,3-butanediol was a suitable solvent.

### b) Effect on the tissue permeability

A test tape preparation was attached to a diced lean beef (about 2 cm cube) and the cube was stood at 4 °C for 24 hours in order to evaluate the tissue permeability of the above tape preparations of lidocaine. The cube was sliced into each 2 mm thick pieces from the attached surface and obtained three fractions (0-2 mm, 2-4 mm and 4-6 mm pieces). Each fraction of pieces was grinded and lidocaine was extracted into methanol. The concentration of lidocaine in methanol was measured by a high-performance liquid chromatography.
This result is shown in Figure 12. This result showed that when propylene carbonate or N-Methyl-2-pyrrolidone was added to a base, a lactic acid salt of lidocaine demonstrated excellent tissue permeability.

### (2) Effect of a solvent on 15 % lidocaine-lactic acid salt

### a) Effect on a transdermal absorbability

According to the procedures described in (1)-a) above, the tape preparations of a lactic acid salt of lidocaine having different solvent compositions (w/w %) as described in the following Table 21 were prepared. Transdermal absorption property was measured according to Test Example 1. In addition, the tissue permeability was measured according to the method described in (1)-a) above.
The results of the transdermal absorbability are also shown in the following Table 21. And, the results of tissue permeability were shown in Figure 13.

**[Table 21]**

| Test preparation | F245-4 | F245-3 | F245-2 | F245-1 |
|---|---|---|---|---|
| lidocaine | 15.0 | 15.0 | 15.0 | 15.0 |
| lactic acid (90.2%) | 6.45 | 6.45 | 6.45 | 6.45 |
| a surfactant: | | | | |
| glycerin | 0.36 | 0.36 | 0.36 | 0.36 |
| monostearate, | 0.64 | 0.64 | 0.64 | 0.64 |
| polyoxyethylene hydrogenated castor oil | | | | |
| solvent: | | | | |
| propylene carbonate, | 0 | 0 | 2.0 | 2.0 |
| 1,3-bunanediol | 0 | 2.0 | 0 | 2.0 |
| softener: | | | | |
| liquid paraffin, | 19.45 | 18.45 | 18.45 | 17.45 |
| Plastibase | 19.0 | 18.0 | 18.0 | 17.0 |
| tackifier: | | | | |
| polybutene, | 1.0 | 1.0 | 1.0 | 1.0 |
| terpene resin | 32.0 | 32.0 | 32.0 | 32.0 |
| elastomer: | | | | |
| SIS | 6.0 | 6.0 | 6.0 | 6.0 |
| antioxidant: | | | | |
| BHT | 0.1 | 0.1 | 0.1 | 0.1 |
| precipitation of crystal | none | none | none | none |
| transdermal absorbability (µg/cm²) | 190 | 169 | 190 | 151 |

The results of the above Table 21 showed that in terms of a transdermal absorbability, propylene carbonate was suitable as a solvent; and in terms of tissue permeability, addition of a mixed solvent of propylene carbonate and 1,3-butanediol could provide more excellent penetrability.
As described above, it was shown that the solvent not only could adjust the lipid solubility of a base but also act as a transdermal promoter and further contribute to the tissue permeability.
Accordingly, it was shown that the proper selections of various solvents involving propylene carbonate and 1,3-butanediol as well as a ratio of their amounts made it possible to produce a tape preparation having an appropriate transdermal absorbability and excellent tissue permeability of a lactic acid salt of lidocaine in a balanced manner.

### (3) Effect of addition of a surfactant

### a) Type of a surfactant

Effects of the presence or absence of a surfactant or its type on a transdermal absorbability were evaluated. According to the procedures described in (1)-a) above, the tape preparations of 30 % lidocaine-lactic acid salt having the compositions (w/w %) which were described in the following Table 22 were prepared. A transdermal absorbability of each tape preparation was evaluated by using Lidoderm as a standard according to Test Example 1. The results are also shown in the following Table 22.

**[Table 22]**

| Test preparation | D270 | D271 | D262 |
|---|---|---|---|
| lidocaine | 30.0 | 30.0 | 30.0 |
| lactic acid (90.2%) | 10.0 | 10.0 | 10.0 |
| surfactant: | -- | HCO-60 | MGS |
| | | 0.4 | 0.4 |
| solvent: | | | |
| 1,3-butanediol | 4.0 | 4.0 | 4.0 |
| a surfactant | -- | HCO-60 | MGS |
| | | 0.4 | 0.4 |
| softener: | | | |
| liquid paraffin | 20.6 | 20.6 | 20.2 |
| tackifier: | | | |
| Arkon P-100 | 24.0 | 24.0 | 24.0 |
| Polybutene | 0.4 | 0.4 | 0.4 |
| elastomer: | | | |
| SIS | 10.0 | 10.0 | 10.0 |
| antioxidant: | | | |
| BHT | 1.0 | 1.0 | 1.0 |
| transdermal absorbability (Lidoderm standard) | 1.2 | 1.8 | 2.5 |

| | | | |
|---|---|---|---|
| [Notes] MGS: Monoglyceride stearate HCO-60: Polyoxyethylene castor oil Lidoderm (Registered Trademark: Endo, lidocaine content 5 %) was used as a standard material. A transdermal absorbability was relatively expressed by using a value of the transdermal absorbability of Lidoderm in a Franz cell as a value 1. | | | |

It was shown from comparison of the test preparations D262 and D270, or the test preparations D271 and D270 in Table 22 above that a transdermal absorbability of lidocaine was improved by the presence of a surfactant.
In addition, it was shown that when MGS was used as a surfactant, a transdermal absorbability of lidocaine was improved by nearly 2-fold than without a surfactant.

### b) Combination of surfactants

The HLB value was adjusted by combining surfactants and the preferable range of the HLB values for a lactic acid salt of lidocaine was examined. According to the procedures described in Example 13-(1), the tape preparations having the following compositions (w/w %) in Table 23 below were prepared. A transdermal absorbability of each tape preparation was evaluated by using a Franz cell according to Test Example 1 and the results are also shown in the following Table 23.

**[Table 23]**

| Test preparation | C 448 | C446 | C 447 |
|---|---|---|---|
| lidocaine | 20.0 | 20.0 | 20.0 |
| lactic acid (90.2%) | 10.4 | 10.4 | 10.4 |
| surfactant: | | | |
| HCO-60 | 2.0 | 1.0 | 0 |
| MGS | 0 | 1.0 | 2.0 |
| (HLB value) | (4) | (8) | (12) |
| solvent: | | | |
| propylene | 4.0 | 4.0 | 4.0 |
| carbonate | | | |
| softener: | | | |
| liquid paraffin | 22.5 | 22.5 | 22.5 |
| tackifier: | | | |
| terpene resin | 28.0 | 28.0 | 28.0 |
| elastomer: | | | |
| SIS | 12.0 | 12.0 | 12.0 |
| antioxidant: | | | |
| BHT | 0.1 | 0.1 | 0.1 |
| transdermal absorbability (µg/cm²) | 54 | 187 | 153 |

| | | | |
|---|---|---|---|
| [Notes] MGS: Monoglyceride stearate HCO-60: Polyoxyethylene castor oil | | | |

The results of the above Table 23 were shown in Figure 14. A transdermal absorbability exhibits a bell-shape curve with a maximum value at a HLB value of around 8 when using a non-ionic surfactant.
Figure 14 showed that in the case that a HLB value of a surfactant was within the range of 6 to 12, an excellent transdermal absorbability was exhibited.

### INDUSTRIAL APPLICABILITY

An external preparation having an excellent transdermal absorbability of a basic pharmacologically active component can be produced by using a method of an external preparation of the present invention. That is, an organic acid salt of a basic pharmacologically active component having an excellent transdermal absorbability can be produced by selecting an appropriate organic acid, based on the combination of the lipid solubility (logP) of the pharmacologically active component and the lipid solubility (logP) of the organic acid. That is, an organic acid salt having an excellent transdermal absorbability can be produced by combining the pharmacologically active component having a lipid solubility of 0.5 to 5 and the organic acid having a lipid solubility of -1 to 4 appropriately.
And then an organic acid salt of a basic pharmacologically active component of the present invention can be dissolved or dispersed into a base of an external preparation by selecting an appropriate solvent and surfactant and an external preparation having the desired dosage form can be made by the base. Thus, the present invention provides an external preparation containing one or more basic pharmacologically active components having the good balance of a transdermal absorbability and tissue permeability.

## Claims

1. A nonaqueous external preparation having an excellent transdermal absorbability comprising an organic acid salt of a basic pharmacologically active component as an active component **characterized by** comprising:
an equimolar salt consisting of a compound having a logP of 0.5 to 5 as a basic pharmacologically active component and a compound having a logP of -1 to 4 as an organic acid; and
an ester solvent and/or an alcohol solvent.

2. The nonaqueous external preparation according to claim 1 wherein the basic pharmacologically active component is one or more selected from the group consisting of tramadol, lidocaine, pentazocine, tolperisone, eperisone, amitriptyline, imipramine, donepezil and morphine.

3. The nonaqueous external preparation according to claim 1 wherein the basic pharmacologically active component is a compound having the logP of 2 to 5.

4. The nonaqueous external preparation according to any of claims 1 to 3 wherein the organic acid is selected from the group consisting of acetic acid, propionic acid, butyric acid, hexanoic acid, glycolic acid, methoxyacetic acid, lactic acid, levulinic acid, benzoic acid, salicylic acid, acetylsalicylic acid and 3-hydroxybutyric acid.

5. The nonaqueous external preparation according to any of claims 1 to 4 wherein the organic acid is a compound having the logP of -1 to 2.

6. The nonaqueous external preparation according to any of claims 1 to 5 wherein the ester solvent is one or more selected from the group consisting of isopropyl myristate, diethyl sebacate, medium-chain fatty acid triglyceride and propylene carbonate.

7. The nonaqueous external preparation according to any of claims 1 to 6 wherein the alcohol solvent is one or more selected from the group consisting of propylene glycol, 2-propanol, 1,3-butanediol and ethylene glycol.

8. The nonaqueous external preparation according to any of claims 1 to 7 wherein the basic pharmacologically active component is lidocaine and the organic acid is lactic acid.

9. The nonaqueous external preparation according to any of claims 1 to 8 wherein the external preparation is a tape preparation.

10. A levulinic acid salt of a basic pharmacologically active component selected from the group consisting of tramadol, lidocaine, pentazocine, tolperisone, eperisone, amitriptyline, imipramine, donepezil and morphine.

11. A lactic acid salt of a basic pharmacologically active component selected from the group consisting of tramadol, lidocaine, pentazocine, tolperisone, eperisone, amitriptyline, imipramine, donepezil and morphine.

12. A method for production of a nonaqueous external preparation comprising a basic pharmacologically active component having an excellent transdermal absorbability which comprises the following steps:
a) selecting a compound having a logP of 0.5 to 5 as a basic pharmacologically active component;
b) selecting a compound having a logP of -1 to 4 as an organic acid to form an equimolecular salt thereof;
c) dissolving the equimolecular salt in an ester solvent and/or an alcohol solvent; and
d) blending and dispersing the above solution into a base of the external preparation.

13. The method for production of the external preparation according to claim 11 wherein the basic pharmacologically active component is one or more selected from the group consisting of tramadol, lidocaine, pentazocine, tolperisone, eperisone, amitriptyline, imipramine, donepezil and morphine.

14. The method for production of the external preparation according to claim 12, wherein the basic pharmacologically active component is a compound having the logP of 2 to 5.

15. The method for production of the external preparation according to any of claims 12 to 14, wherein the organic acid is selected from the group consisting of acetic acid, propionic acid, butyric acid, hexanoic acid, glycolic acid, methoxyacetic acid, lactic acid, levulinic acid, benzoic acid, salicylic acid, acetylsalicylic acid and 3-hydroxybutyric acid.

16. The method for production of the external preparation according to any of claims 12 to 15, wherein the ester solvent is one or more selected from the group consisting of isopropyl myristate, diethyl sebacate and medium-chain fatty acid triglyceride.

17. The method for production of the external preparation according to any of claims 12 to 16, wherein the alcohol solvent is one or more selected from the group consisting of isopropanol, ethylene glycol, propylene glycol and 1,3-butanediol.
